# EUROPÄISCHE PATENTSCHRIFT

(11) **EP 1 682 476 B1**
(45) Veröffentlichungstag und Bekanntmachung des Hinweises auf die Patenterteilung: **06.06.2007**
(21) Anmeldenummer: 04797653.5
(22) Anmeldetag: 05.11.2004
(51) Int. Cl.: C07C 51/09

(54) **VERFAHREN ZUR HERSTELLUNG VON AMEISENSAUREN FORMIATEN**
METHOD FOR THE PRODUCTION OF FORMIC ACID FORMATES
PROCEDE DE PREPARATION DE FORMIATES D'ACIDE FORMIQUE

(30) Priorität: 06.11.2003 DE 10351733; 05.05.2004 DE 102004022135
(43) Veröffentlichungstag der Anmeldung: 26.07.2006
(73) Patentinhaber: BASF Aktiengesellschaft, 67056 Ludwigshafen (DE)
(72) Erfinder: HAUK, Alexander, 67071 Ludwigshafen (DE); KARL, Jörn, 67063 Ludwigshafen (DE); PASCHOLD, Jürgen, 67691 Hochspeyer (DE); GROPP, Stefan, 67346 Speyer (DE); LOHMANN, Anna, Valeska, 67117 Limburgerhof (DE); LENZ, Robert, 67126 Hochdorf-Assenheim (DE); LETZELTER, Thomas, 76855 Annweiler (DE); HEINZ, Robert, 67067 Ludwigshafen (DE)
(74) Vertreter: Isenbruck, Günter
(86) Internationale Anmeldenummer: PCT/EP2004/012543
(87) Internationale Veröffentlichungsnummer: WO 2005/044771

(56) Entgegenhaltungen:
- EP-A- 1 310 172
- WO-A-03/040078
- WO-A-20/04020382
- WO-A-20/04022517

## Beschreibung

Die Erfindung betrifft ein Verfahren zur Herstellung von ameisensauren Formiaten sowie die Verwendung der danach hergestellten ameisensauren Formiate zur Konservierung und/oder Ansäuerung von pflanzlichen und/oder tierischen Stoffen, zur Behandlung von Bioabfällen sowie als Additiv in der Tierernährung oder als Wachstumsförderer für Tiere.

Ameisensaure Formiate besitzen eine antimikrobielle Wirkung und werden beispielsweise zur Konservierung sowie zur Ansäuerung von pflanzlichen und tierischen Stoffen, wie etwa von Gräsern, landwirtschaftlichen Produkten oder Fleisch, zur Behandlung von Bioabfällen oder als Additiv zur Tierernährung eingesetzt.

Als ameisensaure Formiate sind Verbindungen und Gemische zu verstehen, welche Formiat-Anionen (HCOO⁻), Kationen (M^{x+}) und Ameisensäure (HCOOH) enthalten. Sie können zusammen in Form eines Feststoffs oder einer Flüssigkeit vorliegen und gegebenenfalls noch weitere Komponenten, wie beispielsweise weitere Salze, Zusatzstoffe oder Lösungsmittel wie etwa Wasser, enthalten. Im Allgemeinen können die ameisensauren Formiate wiedergegeben werden durch die allgemeine Formel

HCOO⁻M^{x+}_{1/x} * y HCOOH (I),

in der M für ein ein- oder mehrwertiges, anorganisches oder organisches Kation steht, x eine positive ganze Zahl ist und die Ladung des Kations angibt und y den molaren Anteil an Ameisensäure bezogen auf das Formiat-Anion wiedergibt. Der molare Anteil an Ameisensäure bezogen auf das Formiat-Anion y liegt im Allgemeinen bei 0,01 bis 100, bevorzugt bei 0,05 bis 20, besonders bevorzugt bei 0,5 bis 5 und insbesondere bei 0,9 bis 3,1.

Die Natur des anorganischen oder organischen Kations M^{x+} ist prinzipiell unerheblich, sofern dieses unter den Bedingungen, unter denen das ameisensaure Formiat gehandhabt werden soll, stabil ist. Darunter ist beispielsweise auch die Stabilität gegenüber dem reduzierend wirkendem Formiat-Anion zu verstehen. Als mögliche anorganische Kationen seien die ein- und/oder mehrwertigen Metallkationen der Metalle aus der Gruppe 1 bis 14 des Periodensystems, wie beispielsweise Lithium (Li⁺), Natrium (Na⁺), Kalium (K⁺), Cäsium (Cs⁺), Magnesium (Mg²⁺), Kalzium (Ca²⁺), Strontium (Sr²⁺) und Barium (Ba²⁺), bevorzugt Natrium (Na⁺), Kalium (K⁺), Cäsium (Cs⁺) und Kalzium (Ca²⁺) genannt. Als mögliche organische Kationen seien unsubstituiertes Ammonium (NH₄⁺) und durch ein oder mehrere Kohlenstoff enthaltende Reste, welche gegebenenfalls auch miteinander verbunden sein können, substituiertes Ammonium, wie beispielsweise Methylammonium, Dimethylammonium, Trimethylammonium, Ethylammonium, Diethylammonium, Triethylammonium, Pyrollidinium, N-Methylpyrroldinium, Piperidinium, N-Methylpiperidinium oder Pyridinium genannt.

Unter einem Kohlenstoff enthaltenden organischen Rest ist ein unsubstituierter oder substituierter, aliphatischer, aromatischer oder araliphatischer Rest mit 1 bis 30 Kohlenstoffatomen zu verstehen. Dieser Rest kann ein oder mehrere Heteroatome, wie etwa Sauerstoff, Stickstoff, Schwefel oder Phosphor enthalten, beispielsweise -O-, -S-, -NR-, -CO-, -N=, -PR- und/oder -PR₂ und/oder durch eine oder mehrere funktionelle Gruppen, welche beispielsweise Sauerstoff, Stickstoff, Schwefel und/oder Halogen enthalten, substituiert sein, wie beispielsweise durch Fluor, Chlor, Brom, lod und/oder eine Cyanogruppe (bei dem Rest R handelt es sich hierbei ebenfalls um einen Kohlenstoff enthaltenden organischen Rest). Bei dem Kohlenstoff enthaltenden organischen Rest kann es sich um einen einwertigen oder auch mehrwertigen, beispielsweise zwei- oder dreiwertigen Rest handeln.

Zur Herstellung von ameisensauren Formiaten sind eine Vielzahl von Verfahren bekannt. Hierbei wird in der Regel ein flüssiger Strom 1, enthaltend Ameisensäure sowie ein flüssiger Strom 11, enthaltend ein Metallformiat, bereitgestellt und die genannten flüssigen Ströme 1 und 11 unter Erhalt eines ameisensauren Formiat enthaltenden Produktstromes vermischt, der gegebenenfalls weiter aufgearbeitet wird.

In EP-A2 319 172, zum Beispiel, wird die erhaltene Mischung kontinuierlich in eine Destillationskolonne übergeführt. Die aus dem Sumpf der Destillationskolonne abgezogene Metallformiat-AmeisensäureMischung wird auf Lagertemperatur gebracht, wofür ein Wärmetauscher, beispielsweise ein Wirbelbett eingesetzt wird.

Ein derartiges Verfahren ist beispielsweise aus DE-A 102 37 379 bekannt. Danach werden die Ameisensäure bzw. Metallformiat enthaltenden Ströme vorzugsweise in einer Kolonne zusammengebracht, die vorteilhaft so betrieben wird, dass ein Teil des zugeführten Lösungsmittels, in der Regel Wasser, abgezogen wird. Bei dieser Fahrweise kann ein ameisensaures Formiat enthaltendes Sumpfprodukt mit einem Wassergehalt von 0,5 bis 30 Gew.-%, insbesondere mit einem Wassergehalt von im Allgemeinen kleiner oder gleich 1 Gew.-%, erhalten werden.

Demgegenüber war es dem Fachmann bislang trotz der zu erwartenden Vorteile in der Weiterverarbeitung des ameisensaures Formiat enthaltenden Produktstromes nicht bekannt, dass es möglich ist, den Produktstrom mit einem gegenüber bekannten Verfahren wesentlich niedrigeren Wassergehalt unmittelbar aus der Kolonne, in der man den Ameisensäure und den Metallformiat enthaltenden Strom vermischt, zu erhalten.

Entsprechend wurde ein Verfahren zur Herstellung von ameisensauren Formiaten gefunden, wonach man
- einen flüssigen Strom I, enthaltend Ameisensäure und
- einen flüssigen Strom II, enthaltend ein Metallformiat, bereitstellt,
die flüssigen Ströme I und II einer Rektifikationskolonne so zuführt, dass man für den flüssigen Strom II eine höhere oder gleiche Zuführungsstelle zur Rektifikationskolonne als für den flüssigen Strom I wählt,
die flüssigen Ströme I und II in der Rektifikationskolonne unter Abtrennung von Wasser über Kopf der Rektifikationskolonne vermischt und
aus der Rektifikationskolonne einen Sumpfstrom, enthaltend das ameisensaure Formiat, abzieht, das dadurch gekennzeichnet ist, dass man den Sumpfstrom als Schmelze, enthaltend weniger als 0,5 Gew.% Wasser, gewinnt.

In einer bevorzugten Ausgestaltung des Verfahrens stellt man einen flüssigen Strom I, enthaltend Ameisensäure, bereit, der relativ hoch konzentriert ist, d.h. mindestens 85 Gew.-% Ameisensäure enthält. Besonders bevorzugt enthält der flüssige Strom I mindestens 94 Gew.-%, insbesondere 99 Gew.-% Ameisensäure. Vorteilhaft werden hierbei Ameisensäure-Typen eingesetzt, die kommerziell erhältlich sind, beispielsweise Ameisensäure mit einer Reinheit von 85 %, 94 % oder 99 %.

Bei den vorgenannten flüssigen Strömen I, enthaltend Ameisensäure, handelt es sich bevorzugt um wässrige Ströme.

Die Erfinder haben erkannt, dass bei Einsatz von konzentrierten Ameisensäurelösungen ein niedrigerer Restwassergehalt im Zielprodukt, dem ameisensauren Diformiat, das als Schmelze aus der Rektifikationskolonne abgezogen wird, in der die flüssigen Ströme I und II vermischt werden, erhalten werden kann, insbesondere ein Restwassergehalt von unter 0,3 Gew.%, bevorzugt im Bereich von 0,2 bis 0,1 Gew.-% und insbesondere zwischen 0,1 und 0,05 Gew.-%.

Die Erfinder haben auch erkannt, das tendentiell mit der Abnahme des Wassergehaltes im flüssigen Strom I bei ansonsten unveränderten Bedingungen in der Rektifikationskolonne der Wassergehalt in der über Sumpf abgezogenen Schmelze abnimmt.

Der niedrigere Restwassergehalt in der Schmelze beeinflusst entscheidend die Lagerstabilität des formulierten Endproduktes ameisensaures Difomiat: je niedriger der Restwassergehalt im Endprodukt, umso geringer ist die Verbackungs- bzw. Verklumpungsneigung.

Ein weiterer Vorteil im Vergleich zu einer Fahrweise mit höherem Wassergehalt im flüssigen Strom I ist, dass für den Erhalt einer Schmelze mit gleichem, niedrigem Wassergehalt, eine geringere Trennstufenzahl, die insbesondere um etwa 4 bis 8 Trennstufen reduziert ist, ausreicht. Entsprechend kann auch die Rektifikationskolonne mit geringerer Baugröße und somit niedrigeren Investitions- und Betriebskosten ausgeführt werden.

Bevorzugt sind die flüssigen Ströme I und II jeweils wässrige Ströme.

Das Verfahren ist nicht eingeschränkt bezüglich der konkreten Wege zur Bereitstellung der flüssigen Ströme I und II. Bevorzugt können dieselben wie in DE-A 103 21 733 beschrieben zur Verfügung gestellt werden.

Der flüssige Strom I kann beispielsweise durch partielle Hydrolyse von Ameisensäuremethylester, im Folgenden abgekürzt mit MeFo bezeichnet, (Verfahrensstufe (a) aus DE-A 103 21 733) und destillative Abtrennung von nicht umgesetztem MeFo und Methanol erhalten werden.

Der flüssige Strom II kann beispielsweise nach den in DE-A 103 21 733 beschriebenen Verfahrensvarianten erhalten werden:

Danach kann ein MeFo und Methanol umfassender Strom in einer Verfahrensstufe c) durch
i) Umsetzung mit einer basischen Verbindung mit einem pKₐ-Wert der korrespondierenden Säure der entsprechenden Dissoziationsstufe von ≥ 3, gemessen bei 25° in wässriger Lösung, in Gegenwart von Wasser und
ii) destillative Abtrennung des Methanols
in den Metallformiat und Wasser enthaltenden Strom 11 überführt werden.

Zur detaillierten Beschreibung der Verfahrensstufe c) wird auf die genannte DE-A 103 21 733 verwiesen.

In einer weiteren Alternative ist es möglich, den Metallformiat enthaltenden Strom II durch Carbonylierung des entsprechenden Metallhydroxids zu gewinnen. Ein derartiges Verfahren ist in der DE-A 102 37 380 beschrieben.

Im nächsten Verfahrensschritt zur Herstellung der ameisensauren Formiate werden die bereitgestellten Ströme I, enthaltend Ameisensäure und II, enthaltend ein Metallformiat, in einer Rektifikationskolonne vermischt.

Hierbei ist es möglich und gegebenenfalls vorteilhaft, den Ameisensäure enthaltenden flüssigen Strom I und/oder den Metallformiat enthaltenden flüssigen Strom 11 vor dem Vermischen in der Rektifikationskolonne einer Aufkonzentration an Ameisensäure bzw. an Metallformiat zu unterziehen, insbesondere durch Entfernung eines Teils des vorhandenen Wassers durch Verdampfung, bevorzugt durch Abdestillieren.

Es wurde gefunden, dass es für den Betrieb der Rektifikationskolonne wesentlich ist, für den flüssigen Strom II eine höhere oder zumindest gleiche Zuführungsstelle als für den flüssigen Strom I zu wählen. Die Erfinder haben erkannt, dass die Gegenwart von Metallformiat in möglichst weiten Bereichen der Kolonne, insbesondere in Bereichen oberhalb der Zuführung für den Ameisensäure enthaltenden Strom für eine weitgehende Abtrennung des Wassers aus dem Sumpfstrom wichtig ist. Wesentlich ist hierbei, dass das Metallformiat im Stoffsystem in der Kolonne als Schleppmittel für die Ameisensäure fungiert.

Bevorzugt ist ein Betrieb der Rektifikationskolonne bei dem man die Sumpftemperatur in der Rektifikationskolonne auf einen Wert unterhalb von 135°C, insbesondere auf einen Wert unterhalb von 125°C, begrenzt. Hierfür wird der Fachmann unter Berücksichtigung geläufiger Überlegungen, insbesondere des in der Kolonne auftretenden Druckverlustes, den Kopfdruck in der Kolonne entsprechend einstellen.

In einer bevorzugten Verfahrensvariante wählt man die Zuführungsstelle für den flüssigen Strom II auf oder oberhalb der obersten Trennstufe der Rektifikationskolonne mit entsprechend niedrigeren Investitionskosten.

Zusätzlich oder alternativ kann man die Betriebsführung in der Kolonne durch die Wahl der Mengenverhältnisse der flüssigen Ströme II und I beeinflussen: Man kann das Mengenverhältnis der flüssigen Ströme II und I so wählen, dass das Molverhältnis des Metallformiats aus dem flüssigen Strom II und der Ameisensäure aus dem flüssigen Strom I größer als1, kleiner als 1 oder bevorzugt zwischen 0,95 und 1,05, besonders bevorzugt gleich 1 ist. Dadurch kann man die Ameisensäureverluste im Kopfstrom der Rektifikationskolonne begrenzen, ohne dass hierfür ein Verstärkungsteil in der Rektifikationskolonne erforderlich wäre. Der Abzug von nahezu reinem Wasser ist hierbei möglich.

Bei der Auswahl der trennwirksamen Einbauten für die Rektifikationskolonne ist es vorteilhaft, Einbauten mit niedrigem Druckverlust bei gleichzeitig guter Trennleistung, bevorzugt Packungen, zu berücksichtigen.

Die Berechnung der Zahl der theoretischen Trennstufen der Rektifikationskolonne erfolgt nach den allgemeinen fachüblichen Methoden. Für die vorliegende Trennaufgabe dürfte in der Regel eine Anzahl von 5 bis 15 theoretischen Trennstufen bevorzugt sein.

Gegenstand der Erfindung ist auch die Verwendung der nach dem erfindungsgemäßen Verfahren hergestellten ameisensauren Formiate zur Konservierung und/oder Ansäuerung von pflanzlichen und/oder tierischen Stoffen, zur Behandlung von Bioabfällen oder als Additive in der Tierernährung und/oder als Wachstumsförderer für Tiere.

Indem nach dem erfindungsgemäßen Verfahren bereits in der Kolonne, in der die flüssigen Ströme enthaltend Ameisensäure bzw. Metallformiat vermischt werden, der Wertstoff, das ameisensaure Formiat als Schmelze abgezogen wird, mit einem Wassergehalt von weniger als 0,5 Gew.-%, ergeben sich signifikante wirtschaftliche Vorteile: Insbesondere sind für die weitere Aufarbeitung der aus der Rektifikationskolonne abgezogenen Schmelze andere Apparate gegenüber den bekannten Apparaten für die Weiterverarbeitung von Produktströmen mit höherem Wassergehalt erforderlich, die weniger kompliziert und weniger störanfällig sind, insbesondere Kühlwalzen oder - bänder, Kühlplatten oder Prill-Türme. Dadurch sinken die Investitionskosten gegenüber Anlagen für herkömmliche Verfahren mit höherem Wassergehalt im Produktstrom.

Weiterverarbeitungsschritte, wie Fest/Flüssig-Trennungen, Kristallisation und anschließende Trocknung, die bei bekannten Verfahren erforderlich waren, entfallen.

Die Erfindung wird im Folgenden anhand einer Zeichnung sowie von Ausführungsbeispielen näher erläutert.

Es zeigen im Einzelnen:
- Figur 1: die schematische Darstellung einer ersten Ausführungsform einer Anlage zur Durchführung des erfindungsgemäßen Verfahrens und
- Figur 2: die schematische Darstellung einer weiteren bevorzugten Ausführungsform einer Anlage zur Durchführung des erfindungsgemäßen Verfahrens.

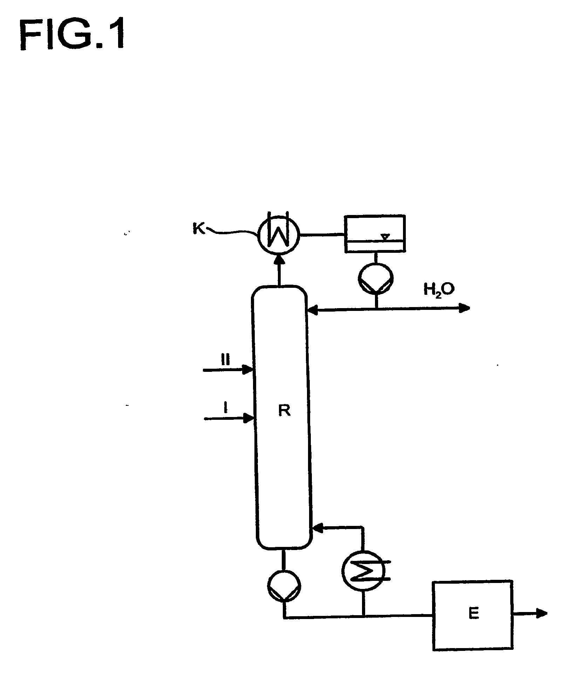

In der in Figur 1 schematisch dargestellten Ausführungsform des erfindungsgemäßen Verfahrens wird eine Rektifizierkolonne R ein wässriger Strom II, enthaltend ein Metallformiat und unterhalb desselben ein wässriger Strom I, enthaltend Ameisensäure, zugeführt. Oberhalb der Zuführung des wässrigen Stromes II ist ein Verstärkungsteil angeordnet. In der Rektifikationskolonne R werden die wässrigen Ströme I und II vermischt und ein überwiegend Wasser enthaltender Kopfstrom abgezogen, der in einem Kondensator K am Kolonnenkopf kondensiert, teilweise als Rücklauf wieder auf die Kolonne aufgegeben und im übrigen ausgeschleust wird. Aus dem Kolonnensumpf wird eine Schmelze, enthaltend weniger als 0,5 Gew.-% Wasser abgezogen und in oder auf einem nachgeschalteten Apparat E zur Erstarrung gebracht. Aus dem nachgeschalteten Apparat E wird, gegebenenfalls nach Behandlung in einem nicht dargestellten Kompaktor das Produkt mit gewünschter Korngröße abgezogen.

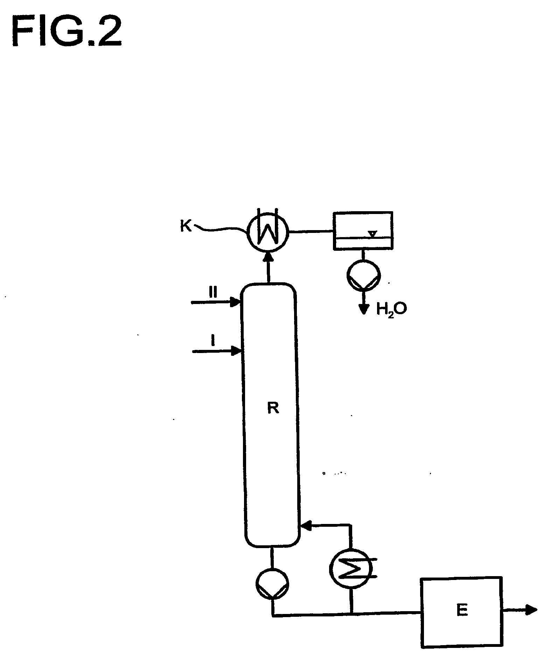

Die in Figur 2 dargestellte bevorzugte Ausführungsform unterscheidet sich von der Ausführungsform in Figur 1 dadurch, dass der wässrige Strom II auf den obersten Boden der Rektifikationskolonne R aufgegeben wird. In dieser Ausführungsform weist somit die Rektifikationskolonne R keinen Verstärkungsteil auf. Auch in dieser Verfahrensvariante kann am Kopf der Rektifikationskolonne weitgehend reines Wasser abgezogen werden, sofern man das Mengenverhältnis der flüssigen Ströme II und 1 so wählt, dass das Molverhältnis des Metallformiats aus dem flüssigen Strom II und der Ameisensäure aus dem flüssigen Strom I größer oder gleich 1 ist.

### Ausführungsbeispiele

In einer Laborkolonne, die aus drei beziehungsweise zwei Schüssen mit einem Durchmesser von jeweils 30 mm zu je 10 Glockenböden bestand, wurden Kaliumdiformiat-Schmelzen erzeugt. Die Kolonne wurde als reine Abtriebskolonne betrieben.

Die Zuläufe bestanden aus 75 %igem wässrigem Kaliumformiat auf dem obersten Boden der Kolonne und aus wässriger Ameisensäure, fünf Böden tiefer. Ameisensäure und Kaliumformiat wurden stöchiometrisch zugefahren.

Ausgetragen wurde im Sumpf Kaliumdiformat mit unterschiedlichen Wassergehalten und am Kopf der Kolonne nahezu reines Wasser mit Resten an Ameisensäure von kleiner als 1000ppm.

In den Beispielen wurde die Ameisensäure-Konzentration im Zulauf variiert. Darüber hinaus wurde bei jeder Ameisensäurekonzentration ein erster Versuch (im Folgenden mit dem Zusatz A gekennzeichnet) bei einem höheren Kopfdruck und ein weiterer Versuch (im Folgenden mit dem Zusatz B gekennzeichnet) bei einem niedrigeren Kopfdruck gefahren.

### Beispiel 1 A

Die Ameisensäure-Konzentration im Zulauf betrug 30 Gew.%. Die Kolonne bestand aus drei Schüssen; der Druckverlust über die Kolonne betrug ca. 35 mbar. Die Kolonne wurde mit einem Kopfdruck von 50 mbar betrieben. Die Sumpftemperaturen lagen zwischen 132 und 135°C. In der Schmelze im Kolonnensumpf wurden Wassergehalte von ca. 0,45 Gew.% erreicht.

### Beispiel 1 B

Die Ameisensäure-Konzentration im Zulauf, die Anzahl der Kolonnenschüsse sowie der Druckverlust über die Kolonne waren gegenüber Beispiel 1 A unverändert. Die Kolonne wurde bei einem Kopfdruck von 20 mbar und einer Sumpftemperatur zwischen 122 und 127°C betrieben. Aus dem Kolonnensumpf wurde eine Schmelze mit einem Wassergehalt von ca. 0,35 Gew.-% abgezogen.

### Beispiel 2 A

Die Ameisensäurekonzentration im Zulauf betrug 85 Gew.-%. Die Kolonne bestand aus zwei Schüssen; der Druckverlust über die Kolonne betrug ca. 25 mbar. Die Kolonne wurde bei einem Kopfdruck von ca. 35 mbar gefahren. Bei einer Sumpftemperatur von etwa 126°C wurden Wassergehalte in der aus dem Kolonnensumpf abgezogenen Kaliumdiformiat-Schmelze zwischen 0,18 und 0,2 Gew.-% erreicht.

### Beispiel 2 B

Die Ameisensäurekonzentration im Zulauf, die Anzahl der Kolonnenschüsse sowie der Druckverlust über die Kolonne waren gegenüber Beispiel 2 A unverändert. Der Kopfdruck wurde auf ca. 25 mbar abgesenkt. Bei einer Sumpftemperatur im Bereich von 124 bis 126°C wurden Wassergehalte in der aus dem Kolonnensumpf angezogenen Kaliumdiformiat-Schmelze von etwa 0,08 bis 0,12 Gew.% erzielt.

### Beispiel 3 A

Die Ameisensäurekonzentration im Zulauf betrug 94 Gew.-%. Die Anzahl der Kolonnenschüsse sowie der Druckverlust entsprachen Beispiel 2 A. Die Kolonne wurde bei einem Kopfdruck von ca. 35 mbar betrieben. Bei einer Sumpftemperatur von 126 bis 128°C wurden Wassergehalte in der aus dem Kolonnensumpf abgezogenden Kaliumdiformiat-Schmelze von etwa 0,08 bis 0,1 Gew.-% erreicht.

### Beispiel 3 B

Die Ameisensäurekonzentration war gegenüber Beispiel 2 A unverändert. Die Anzahl der Kolonnenschüsse sowie der Druckverlust entsprachen Beispiel 2 A. Der Kopfdruck wurde auf ca. 25 mbar abgesenkt. Bei einer Sumpftemperatur von 124 bis 126°C wurde ein Wassergehalt in der aus dem Kolonnensumpf abgezogenen Kaliumdiformiat-Schmelze von etwa 0,05 bis 0,07 Gew.-% erreicht.

### Beispiel 4 A

Die Ameisensäurekonzentration im Zulauf betrug 99 Gew.%. Die Anzahl der Kolonnenschüsse sowie der Druckverlust entsprachen Beispiel 2 A. Die Kolonne wurde bei einem Kopfdruck von ca. 35 mbar betrieben. Bei Sumpftemperaturen von 124 bis 126°C wurden Wassergehalte in der aus dem Kolonnensumpf abgezogenen Kaliumdiformiat-Schmelze von etwa 0,05 bis 0,08 Gew.-% erreicht.

### Beispiel 4 B

Bei unveränderter Ameisensäurekonzentration im Zulauf wurde der Kopfdruck auf ca. 25 mbar abgesenkt. Die Anzahl der Kolonnenschüsse sowie der Druckverlust entsprachen Beispiel 2 A. Die Sumpftemperaturen sowie der Wassergehalt der aus dem Kolonnensumpf abgezogenen Kaliumdiformiat-Schmelze lagen im gleichen Bereich wie unter Beispiel 3 A beschrieben. Somit zeigte sich bei der in den Beispielen 3 A und 3 B eingesetzten hohen Ameisensäurekonzentration kein wesentlicher Einfluss des Kopfdrucks auf den Wassergehalt in der aus dem Kolonnensumpf abgezogenen Schmelze, bei einer Variation des Kopfdrucks von 35 mbar auf 25 mbar.

Die Beispiele 2 A, 2 B, 3 A, 3 B, 4 A und 4 B zeigen, dass in der bevorzugten Verfahrensführung mit hoch konzentrierter Ameisensäure als Eduktstrom I besonders niedrige Wassergehalte in der aus dem Kolonnensumpf abgezogenen Schmelze erreicht werden.

## Patentansprüche

1. Verfahren zur Herstellung von ameisensauren Formiaten, wonach man
- einen flüssigen Strom I, enthaltend Ameisensäure und
- einen flüssigen Strom II, enthaltend ein Metallformiat, bereitstellt,
die flüssigen Ströme I und II einer Rektifikationskolonne so zuführt, dass man für den flüssigen Strom II eine höhere oder gleiche Zuführungsstelle zur Rektifikationskolonne als für den flüssigen Strom 1 wählt,
die flüssigen Ströme I und II in der Rektifikationskolonne unter Abtrennung von Wasser über Kopf der Rektifikationskolonne vermischt und
aus der Rektifikationskolonne einen Sumpfstrom, enthaltend das ameisensaure Formiat, abzieht,
**dadurch gekennzeichnet, dass** man den Sumpfstrom als Schmelze, enthaltend weniger als 0,5 Gew.-% Wasser, gewinnt.

2. Verfahren nach Anspruch 1, **dadurch gekennzeichnet, dass** der Gehalt des flüssigen Stromes I an Ameisensäure mindestens 85 Gew.-% beträgt

3. Verfahren nach Anspruch 2, **dadurch gekennzeichnet, dass** der Gehalt des flüssigen Stromes I an Ameisensäure mindestens 94 Gew.-%, bevorzugt mindestens 99 Gew.-% beträgt.

4. Verfahren nach einem der Ansprüche 1 bis 3, **dadurch gekennzeichnet, dass** die flüssigen Ströme I und II wässrige Ströme sind.

5. Verfahren nach einem der Ansprüche 1 bis 4, **dadurch gekennzeichnet, dass** der Sumpfstrom weniger als 0,3 Gew.-% Wasser, bevorzugt zwischen 0,2 und 0,1 Gew.-% Wasser, besonders bevorzugt 0,1 bis 0,05 Gew.-% Wasser, enthält.

6. Verfahren nach einem der Ansprüche 1 bis 5, **dadurch gekennzeichnet, dass** man die Sumpftemperatur in der Rektifikabonskolonne auf einen Wert unterhalb von 135°C begrenzt.

7. Verfahren nach Anspruch 6, **dadurch gekennzeichnet, dass** man die Sumpftemperatur in der Rektifikationskolonne auf einen Wert unterhalb von 125°C begrenzt.

8. Verfahren nach einem der Ansprüche 1 bis 7, **dadurch gekennzeichnet, dass** man die Zuführungsstelle für den flüssigen Strom II auf oder oberhalb der obersten Trennstufe der Rektifikationskolonne wählt.

9. Verfahren nach einem der Ansprüche 1 bis 8, **dadurch gekennzeichnet, dass** man das Mengenverhältnis der flüssigen Ströme II und I so wählt, dass das Molverhältnis des Metallformiats aus dem flüssigen Strom II und der Ameisensäure aus dem flüssigen Strom I im Bereich von 0,95 bis 1,05, bevorzugt bei 1, liegt.

10. Verfahren nach einem der Ansprüche 1 bis 9, **dadurch gekennzeichnet, dass** man die Rektifikationskolonne mit trennwirksamen Einbauten mit niedrigem Druckverlust, bevorzugt mit Packungen, bestückt

11. Verfahren nach einem der Ansprüche 1 bis 10, **dadurch gekennzeichnet, dass** man die Zahl der theoretischen Trennstufen der Rektikfationskolonne zwischen 5 und 15 wählt.

## Claims

1. A process for preparing acid formates in which
- a liquid stream I comprising formic acid and
- a liquid stream II comprising a metal formate are prepared,
the liquid streams I and II are fed to a rectification column in such a manner that a higher or identical feed point to the rectification column is chosen for the liquid stream II than for the liquid stream I,
the liquid streams I and II are mixed in the rectification column, with water being removed overhead from the rectification column and
a bottoms stream comprising the acid formate is taken off from the rectification column,
which comprises the bottoms stream being produced as melt comprising less than 0.5% by weight of water.

2. The process according to claim 1, wherein the formic acid content of the liquid stream I is at least 85% by weight.

3. The process according to claim 2, wherein the formic acid content of the liquid stream I is at least 94% by weight, preferably at least 99% by weight.

4. The process according to one of claims 1 to 3, wherein the liquid streams I and II are aqueous streams.

5. The process according to one of claims 1 to 4, wherein the bottoms stream comprises less than 0.3% by weight of water, preferably from 0.2 to 0.1% by weight of water, particularly preferably from 0.1 to 0.05% by weight of water.

6. The process according to one of claims 1 to 5, wherein the bottom temperature in the rectification column is limited to a value below 135°C.

7. The process according to claim 6, wherein the bottom temperature in the rectification column is limited to a value below 125°C.

8. The process according to one of claims 1 to 7, wherein the feed point for the liquid stream II is chosen on or above the uppermost separation stage of the rectification column.

9. The process according to one of claims 1 to 8, wherein the ratio of the liquid streams II and I is chosen in such a manner that the molar ratio of metal formate from the liquid stream II and formic acid from the liquid stream I is in the range from 0.95 to 1.05, preferably 1.

10. The process according to one of claims 1 to 9, wherein the rectification column is fitted with separating internals of low pressure drop, preferably with ordered packings.

11. The process according to one of claims 1 to 10, wherein the number of theoretical plates of the rectification column is chosen from 5 to 15.

## Revendications

1. Procédé de préparation de formiates d'acide formique, suivant lequel on prépare :
- un courant liquide I, contenant de l'acide formique, et
- un courant liquide II, contenant un formiate métallique,
on envoie les courants liquides I et II dans une colonne de rectification en choisissant, pour le courant liquide II, un emplacement d'injection plus haut ou à même hauteur, dans la colonne de rectification, que pour le courant liquide I,
on mélange les courants liquides I et II dans la colonne de rectification, avec séparation d'eau en tête de la colonne de rectification, et
on soutire de la colonne de rectification un courant de fond de colonne contenant le formiate d'acide formique,
**caractérisé en ce que** l'on obtient le courant de fond de colonne en tant que masse fondue contenant moins de 0,5% en poids d'eau.

2. Procédé suivant la revendication 1, **caractérisé en ce que** la teneur du courant liquide I en acide formique est d'au moins 85% en poids.

3. Procédé suivant la revendication 2, **caractérisé en ce que** la teneur du courant liquide I en acide formique est d'au moins 94% en poids, de préférence d'au moins 99% en poids;

4. Procédé suivant l'une quelconque des revendications 1 à 3, **caractérisé en ce que** les courants liquides I et II sont des courants aqueux.

5. Procédé suivant l'une quelconque des revendications 1 à 4, **caractérisé en ce que** le courant de fond de colonne contient moins de 0,3% en poids d'eau, de préférence de 0,2 à 0,1% en poids d'eau, plus préférablement de 0,1 à 0,05% en poids d'eau.

6. Procédé suivant l'une quelconque des revendications 1 à 5, **caractérisé en ce qu'**on limite la température du fond de la colonne de rectification à une valeur de moins de 135°C.

7. Procédé suivant la revendication 6, **caractérisé en ce qu'**on limite la température du fond de la colonne de rectification à une valeur de moins de 125°C.

8. Procédé suivant l'une quelconque des revendications 1 à 7, **caractérisé en ce que** l'emplacement d'injection du courant liquide II est choisi sur ou au-dessus du plateau supérieur de la colonne de rectification.

9. Procédé suivant l'une quelconque des revendications 1 à 8, **caractérisé en ce que** l'on choisit le rapport quantitatif des courants liquides II et I de manière à ce que la proportion molaire du formiate métallique du courant liquide II et l'acide formique du courant liquide I soit de l'ordre de 0,95 à 1,05, de préférence 1.

10. Procédé suivant l'une quelconque des revendications 1 à 9, **caractérisé en ce que** l'on équipe la colonne de rectification d'éléments encastrés opérant une séparation avec une faible perte de charge, de préférence de garnissages.

11. Procédé suivant l'une quelconque des revendications 1 à 10, **caractérisé en ce que** l'on choisit le nombre des plateaux théoriques de la colonne de rectification entre 5 et 15.
